(19) Europäisches Patentamt

European Patent Office

Office européen des brevets

(11) **EP 0 861 062 B1**

(12) **EUROPÄISCHE PATENTSCHRIFT**

(45) Veröffentlichungstag und Bekanntmachung des Hinweises auf die Patenterteilung:
**16.08.2001 Patentblatt 2001/33**

(21) Anmeldenummer: **96931778.3**

(22) Anmeldetag: **06.09.1996**

(51) Int Cl.⁷: $A61K\ 6/083$, $A61K\ 6/02$

(86) Internationale Anmeldenummer:
**PCT/EP96/03919**

(87) Internationale Veröffentlichungsnummer:
**WO 97/11670 (03.04.1997 Gazette 1997/15)**

(54) **KIT ZUR HERSTELLUNG EINES DENTALZEMENTS**

KIT FOR THE PRODUCTION OF DENTAL CEMENT

NECESSAIRE POUR PRODUCTION D'UN CIMENT DENTAIRE

(84) Benannte Vertragsstaaten:
**DE**

(30) Priorität: **27.09.1995 DE 19536018**

(43) Veröffentlichungstag der Anmeldung:
**02.09.1998 Patentblatt 1998/36**

(73) Patentinhaber: **DMG - Dental-Material-Gesellschaft mbH 22547 Hamburg (DE)**

(72) Erfinder:
• **WILLMANN, Wolfgang D-25337 Kölln-Reisiek (DE)**

• **BÜNSCH, Almuth D-22395 Hamburg (DE)**

(74) Vertreter: **Glawe, Delfs, Moll & Partner Patentanwälte Rothenbaumchaussee 58 20148 Hamburg (DE)**

(56) Entgegenhaltungen:
**EP-A- 0 232 733      EP-A- 0 261 466
WO-A-91/03987      GB-A- 948 204**

Bemerkungen:
Die Akte enthält technische Angaben, die nach dem Eingang der Anmeldung eingereicht wurden und die nicht in dieser Patentschrift enthalten sind.

**Beschreibung**

Die Erfindung betrifft ein Kit zur Herstellung eines provisorischen dentalen Befestigungszements, einen aus diesem Kit hergestellten Zement, ein Verfahren zu dessen Herstellung, sowie eine mit den Komponenten des Kits gefüllte Zweikomponenten-Austragvorrichtung.

In der Zahnarztpraxis benötigt man provisorische Befestigungsmaterialien für die Befestigung von provisorischen Kronen, Brücken, Inlays etc. Diese Provisorien dienen der Versorgung des Patienten bis zur Fertigstellung des endgültigen Zahnersatzes. Häufig wird auch ein endgültiger Zahnersatz zunächst vorläufig mit einem provisorischen Zement befestigt, um die endgültige Eingliederung und korrekte Paßform zu überprüfen.

Ein für diese Zwecke geeigneter Zement ist aus der US-A-3 028 247 bekannt. Dieser Zement wird aus zwei Pasten zusammengemischt. Die erste Paste enthält Zinkoxid in einer inerten Matrix, die zweite Paste ein Material mit freien Carboxylgruppen.

Die beiden Pasten werden auf einem Mischblock per Hand angemischt. Da die Pasten aufgrund ihres unterschiedlichen rheologischen Verhaltens schlecht miteinander mischbar sind, ist dieser Vorgang aufwendig, eine ausreichende Homogenisierung ist nur schwierig zu erreichen.

GB-A-0 948 204 offenbart in den Beispielen 24 und 25 eine als Zement in der Dentaltechnik verwendbare Zusammensetzung, die Diatomeenerde bzw. pulverisiertes Mica enthält. Dieses Dokument offenbart nicht die speziellen Strukturbildner der vorliegenden Erfindung.

Der Erfindung liegt die Aufgabe zugrunde, ein Kit zur Herstellung eines Dentalzements zu schaffen, aus dem sich einfach, schnell und sicher ein provisorischer Dentalzement mischen läßt. Der erfindungsgemäße Kit enthält folgende Bestandteile:

Komponente 1:
eine erste Paste, die basische Metalloxide gebunden in einer inerten Matrix enthält

Komponente 2:
eine zweite Paste, die enthält:

a) monofunktionelle Carbonsäuren

b) polyfunktionelle Carbonsäuren und/oder Phenole

c) Strukturbildner mit einer spezifischen Oberfläche (BET) von 50 - 400 m$^2$/g ausgewählt aus der Gruppe bestehend aus amorphen Siliciumdioxidmodifikationen, Bentoniten, Zeoliten und Montmorillonit

wobei beide Komponenten ein rheologisches Verhalten aufweisen, das ein Anmischen und Auspressen in einem statischen Mischapplikator in einem Gang erlaubt.

Erfindungsgemäß wird ein Kit geschaffen, in dem beide Komponenten ein rheologisches Verhalten aufweisen, das ein Anmischen und Auspressen in einem statischen Mischapplikator in einem Gang erlaubt. Statische Mischapplikatoren sind im Stand der Technik bekannt und bspw. in EP-A 232 733 und EP-A 261 466 beschrieben. Bei der Anwendung eines solchen Geräts werden beide Komponenten des Kits getrennt in jeweils eine Kartusche eingebracht. Auf jede Kartusche wirkt ein Stempel, mit dessen Hilfe die Pasten ausgepreßt werden. Das Verhältnis der Querschnittsflächen der Kartuschen zueinander entspricht dem gewünschten Mischungsverhältnis der Pasten. Die beiden Pasten treten gemeinsam in eine im genannten Stand der Technik beschriebene statische Mischkanüle ein und werden beim Durchtritt durch die statische Mischkanüle homogen vermischt. Die am Kanülenausgang austretende homogen angemischte Masse kann unmittelbar appliziert werden. Die Verwendung eines solchen statischen Mischapplikators gewährleistet, daß sowohl das Mischungsverhältnis als auch die Mischqualität gleichbleibend gut ist.

Der aus US-A-3 028 247 bekannte Zement läßt sich in einem statischen Mischapplikator nicht anmischen, da der erforderliche Auspreßdruck für die zweite Komponente (polyfunktionelle Carbonsäuren) zu hoch ist. Die Erfindung beruht auf der Erkenntnis, daß sich durch Zusatz eines Strukturbildners zu dieser Paste und entsprechender Verringerung des Anteils polyfunktioneller Carbonsäuren einerseits die rheologischen Eigenschaften dahingehend verändern, daß sich der erforderliche Auspreßdruck beim Anmischen mit einem statischen Mischapplikator verringert und daß überraschenderweise andererseits die mechanischen Eigenschaften des ausgehärteten Zements nicht oder nur unwesentlich verändern, obwohl der Anteil polyfunktioneller Carbonsäuren, die in diesem System den eigentlichen Härter darstellen, deutlich verringert worden ist. Um ein Anmischen und Auspressen in einem statischen Mischapplikator in einem Gang zu erlauben, sollte der Auspreßdruck der Pasten ähnlich sein. Dies bedeutet, daß das Verhältnis der Auspreßdrücke der beiden Pasten zwischen 1:1 und 1:1,8 liegen sollte.

Das Anmischen von Komponente 1 und Komponente 2 erfolgt bevorzugt im Volumenverhältnis 1:10 bis 10:1, weiter bevorzugt 1:3 bis 3:1. Zweckmäßigerweise enthält der Kit die beiden Komponenten von vornherein in dem gewünschten Volumenverhältnis. Bei der Anmischung in einem statischen Mischapplikator wird durch Verwendung von Kartuschen mit entsprechenden Durchmessern automatisch das gewünschte Mischverhältnis eingestellt.

Nachfolgend seien einige im Rahmen der Erfindung verwendete Begriffe erläutert:

Basische Metalloxide sind diejenigen Metalloxide, die bei einer Reaktion mit Wasser Hydroxide bilden oder sich unter Bildung von Basen oder Kationen lösen. Geeignete Metalloxide sind bspw. Zinkoxid, Magnesiumoxid, Titandioxid sowie Calciumhydroxid bzw. -oxid. Zinkoxid ist besonders bevorzugt.

Als inerte Matrix der ersten Paste ist jeder Stoff geeignet, mit dessen Hilfe sich die pulverförmigen Metalloxide zu einer Paste compoundieren lassen, ohne daß die Oxide mit der inerten Matrix reagieren. Als inerte Matrix eignen sich bspw. Paraffine, Vaseline, Weichmacher, Neutralöle, pflanzliche Öle sowie Fettalkohole. Insbesondere eignen sich als inerte Matrix Vaseline, dickflüssiges Paraffin, jeweils nach DAB (Deutsches Arzneimittelbuch) und Pflanzenöle sowie Mischungen dieser Stoffe.

Der Begriff "provisorischer dentaler Befestigungszement" umfaßt alle Zemente des Typs I der ISO 3107 (2. Auflage 1988). Er ist somit beschränkt auf provisorische Zemente für die Befestigung von provisorischen Kronen, Brücken, Inlays etc. Solche provisorischen Befestigungszemente müssen gute Klebeeigenschaften und eine dünne Filmdicke aufweisen. Der Begriff umfaßt nicht provisorische dentale Füllmassen oder sogenannte Cavity Liner (ISO 3107 Typ III und Typ IV). Gegenstand der Erfindung ist somit auch die Verwendung der in den Ansprüchen 1 bis 8 definierten Komponenten zur Herstellung eines provisorischen dentalen Befestigungszements.

Das Mischungsverhältnis von inerter Matrix und basischen Metalloxiden in der ersten Paste muß so bemessen werden, daß sich die gewünschte Pastenkonsistenz einstellt. Der Anteil der inerten Matrix an der ersten Paste beträgt vorzugsweise 10 bis 65 Gew.-%, weiter vorzugsweise 20 bis 55 Gew.-%.

Die in der zweiten Paste enthaltenen monofunktionellen Carbonsäuren sollten wenigstens sechs C-Atome aufweisen, da die niedrigeren Carbonsäuren einen unangenehmen Geruch haben. Bevorzugt ist die Verwendung von Carbonsäuren mit wenigstens zehn C-Atomen. Die Kettenlänge gesättigter Fettsäuren liegt bevorzugt zwischen etwa 10 und 22 C-Atomen. Bei ungesättigten Fettsäuren kann die Kettenlänge ggf. auch etwas höher liegen. Geeignete Fettsäuren sind bspw. Ölsäure, Linolsäure, Linolensäure, Caprylsäure, Caprinsäure, Laurinsäure, Palmitinsäure und Stearinsäure, sowie Mischungen daraus.

Geeignete polyfunktionelle Carbonsäuren bzw. Phenole sind insbesondere Naturharze oder deren Derivate. Naturharze bzw. natürliche Harze sind Harze pflanzlicher oder tierischer Herkunft. Im Rahmen der Erfindung sind unter dem Begriff Naturharze auch synthetisch hergestellte Harze zu verstehen, die gleiche oder ähnliche Eigenschaften wie natürliche Harze aufweisen.

Grundsätzlich sind alle Harze geeignet, die der phänomenologischen Definition der DIN 55958 unterfallen und freie Phenol- oder Carboxygruppen aufweisen. Eine Übersicht über verschiedene natürliche und synthetische Harze findet sich in Ullmanns Encyclopaedia of Industrial Chemistry, 5. Auflage, Band A23, Seite 73 bis 110.

Geeignete Harze sind bspw. Copal- und Dammarharze (siehe Ullmann, Supra, Seiten 76 bis 77) sowie Kolophonium (siehe Definition in Römpp Chemielexikon, 9. Auflage) und deren Derivate.

Die Strukturbildner in der zweiten Paste weisen eine spezifische Oberfläche (BET) von 50 - 400 $\frac{m^2}{g}$ auf und werden ausgewählt aus der Gruppe bestehend aus amorphen Siliciumdioxidmodifikationen, Bentoniten, Zeoliten und Monmorillonit. Das thixotrope Verhalten von Pasten mit diesen Strukturbildnern ist gleichmäßiger und verändert sich auch bei länger dauernder Lagerung der Paste nur wenig. Der Anteil der Strukturbildner an der zweiten Paste liegt bevorzugt zwischen 10 und 70 Gew.-%, weiter vorzugsweise zwischen 20 und 40 Gew.-%.

Als Strukturbildner in der zweiten Paste sind amorphe Siliciumdioxidmodifikationen, wie beispielsweise pyrogene und gefällte Kieselsäuren besonders geeignet. Andere erfindungsgemäße Strukturbildner sind Bentonite, Zeolite und Monmorillonit. Das thixotrope Verhalten von Pasten mit diesen Strukturbildnern ist gleichmäßig und verändert sich auch bei länger dauernder Lagerung der Paste nur wenig. Der Anteil der Strukturbildner an der zweiten Paste liegt bevorzugt zwischen 10 und 70 Gew.-%, weiter vorzugsweise zwischen 20 und 40 Gew.-%.

Nachfolgend werden Ausführungsbeispiele der Erfindung und Vergleichsbeispiele erläutert. In der Zeichnung zeigen:

Fig. 1     eine Fließkurve der zweiten Paste des Beispiels 1;

Fig. 2     eine Fließkurve der zweiten Paste des Vergleichsbeispiels 1.

Fig. 3     einen teilweisen Längsschnitt durch eine erfindungsgemäße Zweikomponenten-Austragvorrichtung;

Fig. 4     eine Ansicht dieser Vorrichtung von der Mündungsseite her;

Fig. 5     den statischen Mischapplikator von der Eintrittsseite her.

### Beispiel 1

Die beiden Komponenten bzw. Pasten eines Kits werden aus folgenden Bestandteilen homogen angemischt:

| Komponente 1: | |
|---|---|
| Paraffin, dickflüssig nach DAB | 40 Gew.-% |
| ZnO | 30 Gew.-% |
| MgO | 25 Gew.-% |
| Ca(OH)$_2$ | 5 Gew.-% |

| Komponente 2: | |
|---|---|
| Caprinsäure | 30g |
| Kolophonium | 45g |
| Aerosil R 202® | 24g |

Aerosil R 202® ist ein amorphes hydrophiles Siliciumdioxid, das von der Degussa AG vertrieben wird. Die spezifische Oberfläche (BET) beträgt $100 \pm 20$ m$^2$/g (Stickstoff-Adsorption).

Die beiden Pasten werden getrennt in die Kammern einer Doppelkartusche entsprechend EP-A 261 466 gefüllt, die Doppelkartusche wird mit einer statischen Mischkanüle versehen und die Pasten werden unter Verwendung einer Austragvorrichtung entsprechend EP-A 232 733 durch die statische Mischkanüle hindurch appliziert. Das Volumenverhältnis, in dem die beiden Pasten angemischt werden, beträgt 1:1. Das Mischresultat nach Durchtritt durch die statische Mischkanüle ist sehr homogen.

Beispiel 2

| Komponente 1: | |
|---|---|
| Olivenöl | 10 Gew.-% |
| Paraffine, dickflüssig nach DAB | 10 Gew.-% |
| Ca(OH)$_2$ | 10 Gew.-% |
| ZnO | 70 Gew.-% |

| Komponente 2: | |
|---|---|
| Laurinsäure | 25 g |
| Dammar-Harz | 40 g |
| Aerosil R 202® | 3 g |
| Hydroxylapatit | 34 g |

Diese Pasten lassen sich 1:1 (v/v) mit einem statischen Mischer wie vorstehend beschrieben anmischen. Der Auspreßdruck der Komponente 1 beträgt 1,0 N/mm$^2$ und der der Komponente 2 1,6 N/mm$^2$.

Beispiel 3

| Komponente 1: | |
|---|---|
| Vaseline nach DAB | 50 Gew.-% |
| MgO | 25 Gew.-% |
| ZnO | 25 Gew.-% |

| Komponente 2: | |
|---|---|
| Fettsäuren | 30 g |
| Copal-Harz | 45 g |
| Paraffin | 5 g |
| Aerosil R 202® | 24 g |

Diese Pasten lassen sich leicht 2:1 (v/v) mit einem statischen Mischer anmischen.

Vergleichsbeispiel 1 (nicht erfindungsgemäß)

| Komponente 1: | |
|---|---|
| Paraffin, dickflüssig, nach DAB | 50 Gew.-% |
| ZnO | 50 Gew.-% |

| Komponente 2: | |
|---|---|
| Fettsäuren (gesättigt, C$_{16}$-C$_{20}$) | 30 g |
| Kolophonium | 70 g |

Diese Pasten lassen sich getrennt in eine Doppelkartusche eingefüllt mittels eines statischen Mischapplikators nicht auspressen.

Vergleichsbeispiel 2 (nicht erfindungsgemäß)

| Komponente 1: | |
|---|---|
| Olivenöl | 11,3 g |
| ZnO | 40 g |

| Komponente 2: | |
|---|---|
| Pelargonsäuren | 40 g |
| Ölsäure | 28 g |
| Kolophonium-Glycerolester | 52 g |

Diese Pasten lassen sich getrennt in eine Doppelkartusche eingefüllt mittels eines statischen Mischapplikators nicht auspressen.

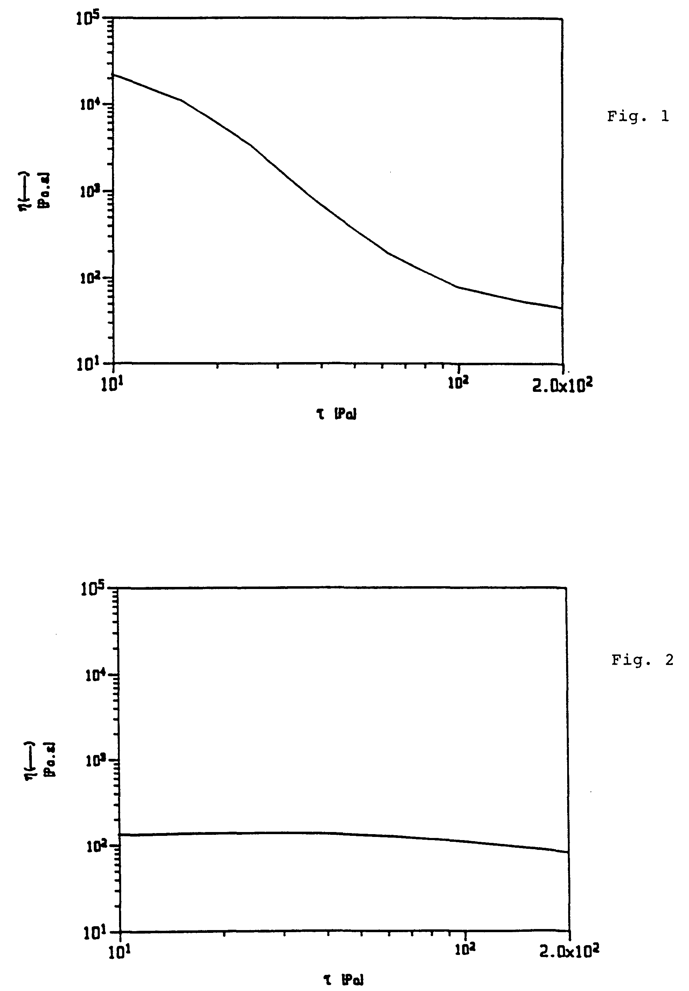

Die rheologischen Unterschiede der Komponente 2 des Beispiels 1 sowie des Vergleichsbeispiels 1 sind aus den Fließkurven der Zeichnung ersichtlich. Diese Fließkurven sind mit dem Rheometer DSR der Fa. Rheometrix aufgenommen worden. Die Meßparameter sind: Plattendurchmesser 25 mm, Spaltbreite 1 mm, Temperatur 23°C, Anfangsstress 10 Pa, Endstress 200 Pa. In den Fig. ist jeweils die Viskosität η gemessen in Pa · s gegen die Schubspannung τ gemessen in Pa aufgetragen. Man erkennt, daß die Paste gemäß Beispiel 1 thixotropes Verhalten aufweist, also bei niedrigerer Schubspannung eine hohe Viskosität und damit ein hohes Standvermögen aufweist und bei hoher Schubspannung eine niedrigere Viskosität und damit eine gute Fließfähigkeit aufweist. Die Komponente 2 gemäß Vergleichsbeispiel 1 weist kein thixotropes Verhalten auf (siehe Fig. 2).

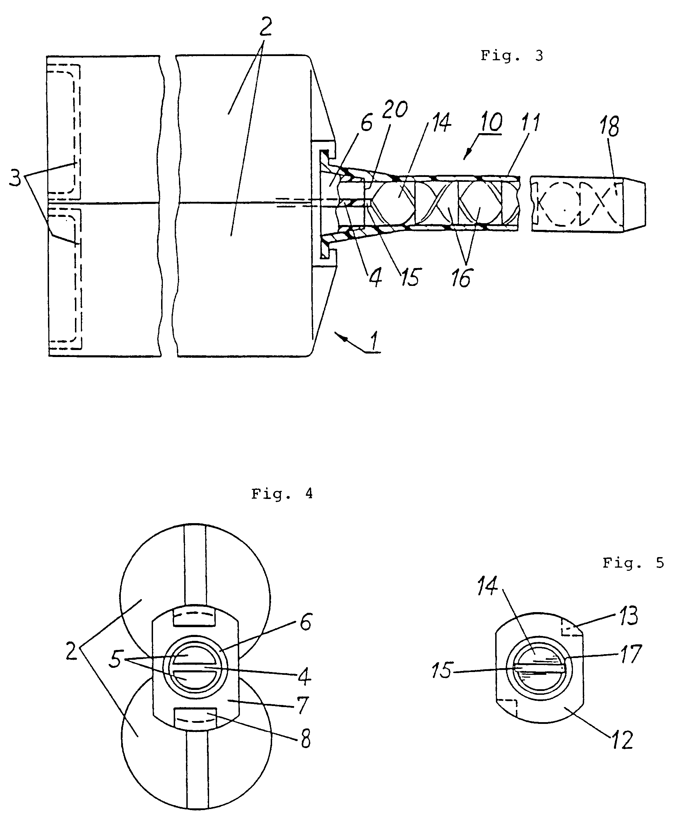

Gegenstand der Erfindung ist ferner eine Zweikomponenten-Austragvorrichtung, die fertig mit den Komponenten eines erfindungsgemäßen Kits befüllt ist. Ein Ausführungsbeispiel ist in den Fig. 3 bis 5 gezeigt. Diese Austragvorrichtung ist für sich genommen aus EP-A-0 232 733 bekannt.

Die Zweikomponenten- oder Doppelkartusche 1 der in Fig. 3 dargestellten Austragvorrichtung weist zwei nebeneinanderliegende, zylindrische Vorratsbehälter 2 auf, die je mit einem Förderkolben 3 für den Austrag des Kartuscheninhalts versehen sind. Aus dem Vorratsraum in jedem Zylinder 2 führt je ein Austragkanal 5 in einem gemeinsamen Mündungsteil 6 der Kartusche, wobei die Kanäle 5 bis zur Mündung durch eine Wand 4 getrennt sind. Zur lösbaren Befestigung eines an die Kartuschenmündung anschließenden Strömungsmischers oder statischen Mischapplikators 10 weist die Kartusche 1 eine den Mündungsteil 6 umgebende Sockelplatte 7 auf, die zur Halterung des Mischers nach Art eines Bajonettverschlusses zwei gegenüberliegende Halteklauen 8 aufweist.

Der Strömungsmischer 10 (auch "statischer Mischer" genannt) weist ein Mischerrohr II auf, welches eingangsseitig über den Mündungsteil 6 passend konisch ausgebildet und mit einem Flansch 12 versehen ist. Beim Anschließen des Mischers an die Kartusche wird der Flansch 12 in gegenüber Fig. 3 um 90° versetzter Drehlage zwischen den Klauen 8 auf die Sockelplatte 7 gesetzt und dann um eine Vierteldrehung gedreht, wobei der Flansch unter die Klauen 8 greift. Durch zwei auf dem Flansch 12 vorgesehene Anschläge 13, die an den Klauen 8 zur Anlage kommen, ist die Drehlage des angeschlossenen Mischers 10 gegenüber der Kartusche bestimmt.

Im Rohr 11 des Strömungsmischers befinden sich längs aneinandergereiht eine Anzahl abwechselnd rechts- und linksdrehend gewundene Mischflügel 14, 16, die jeweils in der Drehlage gegeneinander um 90° versetzt sind. Bei der vorliegenden Mischerbauart ist vorzugsweise die Gesamtheit der Mischflügel 14, 16 einteilig als Kunststoff-Spritzteil gefertigt und als Mischelement insgesamt im Mischerrohr 11 gehalten, jedoch ist auch eine Gruppierung der Flügel zu zwei oder mehreren Teilelementen möglich.

Der erste Flügel 14 des Mischers ist so im Mischerrohr 11 gehalten, daß seine Eintrittskante 15 beim beschriebenen Anschluß des Mischers 10 an die Doppelkartusche 1 zur Trennwand 4 der Kartuschenmündung parallel liegt und an diese dicht anschließt. Dadurch bildet der erste Flügel 14 eine - wenn auch gewundene - Fortsetzung der Trennwand 4 in das Mischerrohr 11 hinein, d.h. die Austragkanäle 5 im Mündungsteil 6 der Kartusche werden über die Anschlußteile 20 des Mischers hinaus getrennt weitergeführt. Die bei Betätigung der Austragvorrichtung durch die beiden Kanäle 5 austretenden Komponentenströme treffen somit nicht bereits beim Verlassen der Kartuschenmündung aufeinander, sondern erstmals beim Übergang vom ersten Flügel 14 zum nachfolgenden Flügel 16. Damit ist gewährleistet, daß auch bei längerem Liegenlassen der Austragvorrichtung nach begonnenem Austrag im Anschlußbereich des Mischers (Stelle 20) und vor allem in der Kartuschenmündung keine Reaktion bzw. Verhärtung des Kartuscheninhalts stattfindet, d.h. die Aushärtung oder "Knollenbildung" erfolgt ausschließlich weiter vorn (in Strömungsrichtung) im ohnehin nicht weiter verwendbaren Mischer.

Die erforderliche Relativlage des ersten Flügels 14 im Mischerrohr 11 (und damit natürlich auch der nachfolgenden Flügel 16) kann lediglich durch Haftsitz, d.h. Abstimmung der Durchmesser des Flügels und der Rohr-Bohrung sichergestellt werden. Beim dargestellten Ausführungsbeispiel ist dagegen der Flügel 14 im Bereich der Eintrittskante 15 in Richtung des Rohrdurchmessers etwas verlängert und in entsprechenden kleinen Ausnehmungen 17 in der Rohrwand gehalten (Fig. 3). Auf diese Weise kann auch eine für den dichten Anschluß empfehlenswerte axiale Anpressung der Kante 15 an die Stirnseite der Trennwand 4 leicht erreicht werden. Die Eintrittskante 15 kann mit Vorteil auch schneidenartig gestaltet werden. Wenn, wie üblich, sämtliche Flügel 14, 16 ein einteiliges Mischelement (z. B. ein Kunststoff-Spritzteil) bilden, ergibt sich die weitere Möglichkeit, das Mischelement am Austrittsende 18 gegenüber dem Rohr 11 axial und in der Drehlage zu positionieren, z. B. mittels nockenartiger Führungen an der Innenwand des Rohrs 11 (nicht dargestellt), um den richtigen Anschluß der Eintrittskante 15 sicherzustellen; durch geeignete Längenbemessung von Mischelement und Rohr läßt sich dann auch ein federnd dichtes Anliegen der Eintrittskante 15 durch leichtes axiales Zusammendrücken des Mischelements beim Aufsetzen des Mischers 10 auf die Kartusche 1 erreichen.

Erfindungsgemäß ist vorgesehen, daß in den beiden Vorratsräumen 2 jeweils eine Komponente eines erfindungsgemäßen Kits eingefüllt ist. Die erfindungsgemäße Austragvorrichtung wird somit fertig befüllt in den Handel gebracht und kann vom Anwender sofort verwendet werden. Zeitaufwendige Vorbereitungshandlungen entfallen. Der Anwender kann mit der erfindungsgemäßen Austragvorrichtung gewissermaßen in situ die gerade benötigte Menge eines provisorischen dentalen Befestigungszements anmischen und applizieren.

**Patentansprüche**

1. Kit zur Herstellung eines provisorischen dentalen Befestigungszements, dadurch gekennzeichnet, daß er folgende Bestandteile enthält:

   Komponente 1:
   eine erste Paste, die basische Metalloxide gebunden in einer inerten Matrix enthält

   Komponente 2:
   eine zweite Paste, die enthält:

   a) monofunktionelle Carbonsäuren
   b) polyfunktionelle Carbonsäuren und/oder Phenole
   c) Strukturbildner mit einer spezifischen Oberfläche (BET) von 50 - 400 $m^2/g$ ausgewählt aus der Gruppe bestehend aus amorphen Siliciumdioxidmodifikationen, Bentoniten, Zeoliten und Montmorillonit

   wobei beide Komponenten ein rheologisches Verhalten aufweisen, das ein Anmischen und Auspressen in einem statischen Mischapplikator in einem Gang erlaubt.

2. Kit nach Anspruch 1, dadurch gekennzeichnet, daß die erste Paste Zinkoxid enthält.

3. Kit nach Anspruch 1 oder 2, dadurch gekennzeichnet, daß als Strukturbildner amorphe Kieselsäuren verwendet werden.

4. Kit nach einem der Ansprüche 1 bis 3, dadurch gekennzeichnet, daß die zweite Paste monofunktionelle Carbonsäuren mit wenigstens 6 C-Atomen enthält.

5. Kit nach Anspruch 4, dadurch gekennzeichnet, daß die monofunktionellen Carbonsäuren Fettsäuren sind.

6. Kit nach einem der Ansprüche 1 bis 5, dadurch gekennzeichnet, daß die zweite Paste Naturharze und/oder deren Derivate enthält.

7. Kit nach einem der Ansprüche 1 bis 6, dadurch gekennzeichnet, daß das Mengenverhältnis von Komponente 1 zu Komponente 2 in dem Kit zwischen 1:

10 und 10:1 (v/v) liegt.

8. Kit nach Anspruch 7, dadurch gekennzeichnet, daß dieses Mengenverhältnis zwischen 1:3 und 3:1 (v/v) liegt.

9. Zement, dadurch gekennzeichnet, daß er aus den Komponenten eines Kits gemäß einem der Ansprüche 1 bis 8 hergestellt ist.

10. Verfahren zur Herstellung eines Zements, gekennzeichnet durch folgende Schritte:

    - die Komponenten eines Kits gemäß einem der Ansprüche 1 bis 8 werden in einen statischen Mischapplikator eingebracht

    - die Komponenten werden durch eine statische Mischkanüle ausgepreßt und dabei in der Mischkanüle vermischt.

11. Verfahren zur Herstellung eines Zements nach Anspruch 10, dadurch gekennzeichnet, daß Komponente 1 und Komponente 2 des Kits im Mengenverhältnis 1:10 bis 10:1 (v/v) vermischt werden.

12. Verfahren zur Herstellung eines Zements nach Anspruch 11, dadurch gekennzeichnet, daß dieses Mengenverhältnis zwischen 1:3 und 3:1 (v/v) liegt.

13. Zweikomponenten-Austragvorrichtung, mit einer Austragkartusche (1) mit zwei je aus einem Vorratsraum (2) in einen gemeinsamen Mündungsteil (6) führenden Austragkanälen (5), sowie einen an die Mündung anschließenden statischen Mischapplikator (10) mit in einem Mischerrohr (11) angeordneten Mischflügeln (14, 16, 18), dadurch gekennzeichnet, daß in einem Vorratsraum (2) die Komponente 1 und in dem anderen Vorratsraum (2) die Komponente 2 eines Kits gemäß einem der Ansprüche 1 bis 8 angeordnet sind.

**Claims**

1. Kit for preparing a provisional dental fixing cement, characterized in that it comprises the following components:

   component 1:
   a first paste, comprising basic metal oxides bound in an inert matrix
   component 2:
   a second paste, comprising:

   a) monofunctional carboxylic acids
   b) polyfunctional carboxylic acids and/or phenols

c) structuring agents having a specific surface area (BET) of 50-400 m$^2$/g selected from the group consisting of amorphous silica modifications, bentonites, zeolites and montmorillonite

both components possessing a rheology that permits mixing and expression in a static mixer-applicator in one operation.

2. Kit according to Claim 1, characterized in that the first paste comprises zinc oxide.

3. Kit according to Claim 1 or 2, characterized in that amorphous silicas are used as structuring agents.

4. Kit according to one of Claims 1 to 3, characterized in that the second paste comprises monofunctional carboxylic acids having at least 6 carbon atoms.

5. Kit according to Claim 4, characterized in that the monofunctional carboxylic acids are fatty acids.

6. Kit according to one of Claims 1 to 5, characterized in that the second paste comprises natural resins and/or their derivatives.

7. Kit according to one of Claims 1 to 6, characterized in that the ratio of component 1 to component 2 in the kit is between 1:10 and 10:1 (v/v).

8. Kit according to Claim 7, characterized in that the said ratio is between 1:3 and 3:1 (v/v).

9. Cement, characterized in that it is produced from the components of a kit according to one of Claims 1 to 8.

10. Process for producing a cement, characterized by the following steps:

- the components of a kit according to one of Claims 1 to 8 are introduced into a static mixer-applicator
- the components are expressed through a static hollow mixing needle in which they are at the same time mixed.

11. Process for producing a cement, according to Claim 10, characterized in that component 1 and component 2 of the kit are mixed in a ratio of from 1:10 to 10:1 (v/v).

12. Process for producing a cement, according to Claim 11, characterized in that the said ratio is between 1:3 and 3:1 (v/v).

13. Two-component discharge apparatus, having a discharge cartridge (1) with two discharge ducts (5) each leading from a storage chamber (2) into a common outlet section (6), and having a static mixer-applicator (10) which adjoins the outlet and has mixing paddles (14, 16, 18) disposed in a mixer pipe (11), characterized in that component 1 of a kit according to one of Claims 1 to 8 is disposed in one storage chamber (2) and component 2 is disposed in the other storage chamber (2).

**Revendications**

1. Kit pour la fabrication d'un ciment dentaire provisoire de fixation, caractérisé en ce qu'il contient les constituants suivants:

composant (1):
une première pâte qui contient des oxydes métalliques basiques liés dans une matrice inerte

composant (2):
une deuxième pâte qui contient:

a) des acides carboniques monofonctionnels

b) des acides carboniques polyfonctionnels et/ou des phénols

c) des structurants avec une surface spécifique (BET) de 50 à 400 m$^2$/g, choisis dans le groupe constitué de modifications amorphes de dioxyde de silicium, de bentonites, de zéolithes et de montmorillonite,

les deux composants présentant un comportement rhéologique qui permet un mélange et une injection en une opération, dans un applicateur statique de mélange.

2. Kit selon la revendication 1, caractérisé en ce que la première pâte contient un oxyde de zinc.

3. Kit selon la revendication 1 ou 2, caractérisé en ce qu'on utilise comme structurants des acides siliciques amorphes.

4. Kit selon l'une des revendications 1 à 3, caractérisé en ce que la deuxième pâte contient des acides carboniques monofonctionnels avec au moins 6 atomes de C.

5. Kit selon la revendication 4, caractérisé en ce que les acides carboniques monofonctionnels sont des acides gras.

6. Kit selon l'une des revendications 1 à 5, caractérisé en ce que la deuxième pâte contient des résines naturelles et/ou leurs dérivés.

7. Kit selon l'une des revendications 1 à 6, caractérisé en ce que le rapport en quantité du composant (1) au composant (2) dans le kit se situe entre 1 à 10 et 10 à 1 (v/v).

8. Kit selon la revendication 7, caractérisé en ce que ce rapport en quantité se situe entre 1 à 3 et 3 à 1 (v/v).

9. Ciment, caractérisé en ce qu'il est préparé à partir des composants d'un kit selon l'une des revendications 1 à 8.

10. Procédé de préparation d'un ciment, caractérisé par les étapes suivantes :

- les composants d'un kit selon l'une des revendications 1 à 8 sont introduits dans un applicateur statique de mélange,

- les composants sont injectés au moyen d'une canule statique de mélange et pendant ce temps mélangés dans la canule de mélange.

11. Procédé de préparation d'un ciment selon la revendication 10, caractérisé en ce que le composant (1) et le composant (2) du kit sont mélangés dans un rapport en quantité de 1 à 10 à 10 à 1 (v/v).

12. Procédé de préparation d'un ciment selon la revendication 11, caractérisé en ce que ce rapport en quantité est compris entre 1 à 3 et 3 à 1 (v/v).

13. Dispositif de distribution de deux composants, comportant une cartouche de distribution (1) avec deux canaux de distribution qui mènent chacun d'une chambre de réserve (2) à un embout (6) commun, ainsi qu'un applicateur statique de mélange (10), se raccordant à l'embouchure, avec des pales de mélange (14, 16, 18) disposées dans un tube mélangeur (11), caractérisé en ce que dans une chambre de réserve (2) est placé le composant (1) et dans l'autre chambre de réserve (2), le composant (2) d'un kit selon l'une des revendications 1 à 8.

Fig. 1

Fig. 2

9

Fig. 3

Fig. 4

Fig. 5